Europäisches Patentamt

(19) European Patent Office

Office européen des brevets

(11) Publication number: **0 029 309**
**B1**

(12) **EUROPEAN PATENT SPECIFICATION**

(45) Date of publication of patent specification: **11.04.84**

(51) Int. Cl.³: **A 61 K  35/66,** C 1 2 P  1/06, C 0 7 G  1 1/00 // C12R1/465

(21) Application number: **80303807.4**

(22) Date of filing: **27.10.80**

(54) **Substances having antibiotic activity, processes for their preparation, pharmaceutical compositions containing them, their use in medicaments and microorganisms.**

(30) Priority: **26.10.79 JP 137734/79**
**13.05.80 JP 137734/79**

(43) Date of publication of application:
**27.05.81 Bulletin 81/21**

(45) Publication of the grant of the patent:
**11.04.84 Bulletin 84/15**

(84) Designated Contracting States:
**DE FR GB IT**

(56) References cited:
**GB - A - 1 113 501**

(73) Proprietor: **KYOWA HAKKO KOGYO CO., LTD**
**Ohtemachi Bldg. Ohtemachi 1-chome Chiyoda-ku Tokyo (JP)**

(72) Inventor: **Tomita, Fusao**
**1420-18, Honmachida**
**Machida-shi Tokyo-to (JP)**
Inventor: **Tamaoki, Tatsuya**
**3-9-9, Naka-machi**
**Machida-shi Tokyo-to (JP)**
Inventor: **Shirahata, Kunikatsu**
**1-12-2, Asahi-machi**
**Machida-shi Tokyo-to (JP)**
Inventor: **Iida, Takao**
**2-18-3, Shimo**
**Kita-ku Tokyo-to (JP)**
Inventor: **Morimoto, Makoto**
**13-9, Miyuki-cho**
**Numazu-shi Shizuoka-ken (JP)**
Inventor: **Fujimoto, Kazuhisa**
**1188, Shimo-togari Nagaizumi-cho**
**Sunto-gun Shizuoka-ken (JP)**

(74) Representative: **Watkins, Arnold Jack et al,**
**European Patent Attorney Frank B. Dehn & Co.**
**Imperial House 15-19 Kingsway**
**London WC2B 6UZ (GB)**

Courier Press, Leamington Spa, England.

Substances having antibiotic activity, processes for their preparation, pharmaceutical compositions containing them and their use as medicaments

The present invention relates to substances having antibiotic activity, processes for their preparation, pharmaceutical compositions containing them and their use as medicaments. The substances of the present invention have been designated DC—45.

According to one feature of the present invention there is provided a substance having antibiotic activity selected from substances designated DC—45—A, DC—45—$B_1$ and DC—45—$B_2$ and respectively possessing the following physico-chemical characteristics:

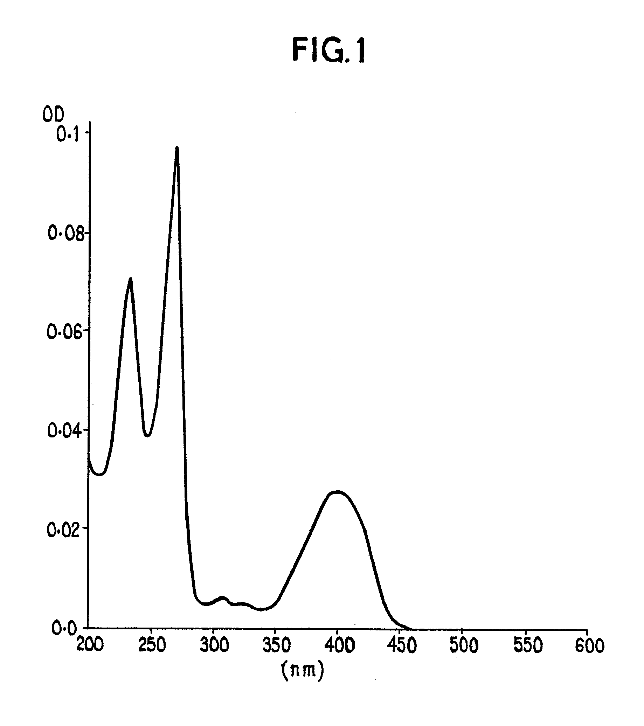

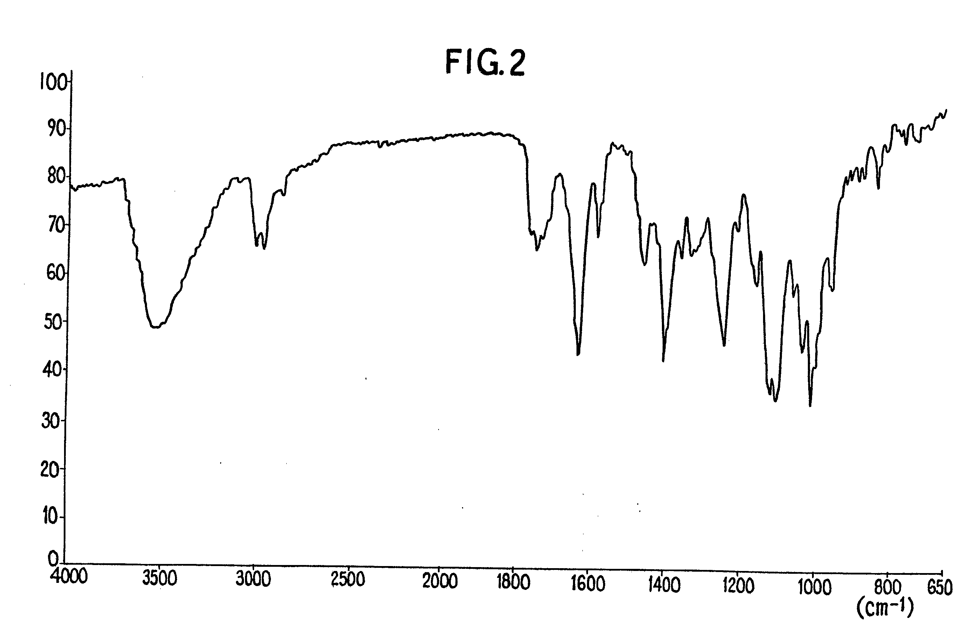

DC—45—A:
(1) Elemental analysis: C:55.11%, H:5.74%
(2) Molecular weight: 877
(3) Melting point:
180±3°C (decomp.)
(4) Ultraviolet absorption spectrum:
As shown in Figure 1 (in 50% methanol)
(5) Infrared absorption spectrum:
As shown in Figure 2 (KBr tablet method)
(6) Specific rotation:
$[\alpha]_D^{25}=-15.3°$ (c=1.0, ethanol)
(7) PMR spectrum (in $CDCl_3$; ppm):
1.07 (3H, s); 1.10 (3H, d, J=6.8); 1.24 (3H, d, J=6.5); many peaks are found between 1.40—2.30; 2.14 (3H, s); 2.49 (3H, s); 2.63 (3H, s); many peaks are found between 2.30—2.80; 2.91 (1H, d, J=5.6); 3.00 (1H, d, J=5.6); 3.49 (3H, s); 3.63 (3H, s); 3.85 (3H, s); many peaks are found between 3.60—4.00; 4.18 (1H, s); 4.55 (1H, q, J=6.8); many peaks are found between 4.70—4.90; 5.03 (1H, q, J=6.5); 5.25 (1H, d, J=4.0); 5.39 (1H, d, J=4.0); 5.87 (1H, m); 7.52 (1H, s); 14.1 (1H, s)
(8) CMR spectrum (in $CDCl_3$; ppm):
210.3; 203.0; 170.2; 163.1; 151.7; 144.7; 142.8; 135.4; 126.6; 116.9; 114.8; 107.5; 104.6; 101.5; 99.7; 98.0; 94.8; 79.5; 74.4; 70.2; 69.1; 68.8; 68.3; 67.9; 67.6; 63.8; 62.9; 62.7; 56.7; 56.2; 48.1; 36.7; 31.5; 27.7; 25.7; 20.8; 20.3; 16.8; 14.5.
(9) Solubility:
Soluble in methanol, ethanol, acetone, ethyl acetate and chloroform, soluble with difficulty in benzene, ether and water, and insoluble in n-hexane.

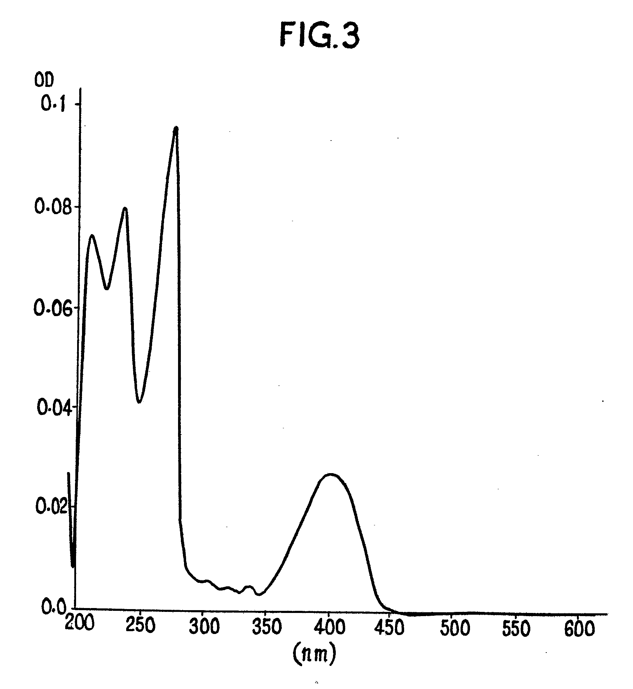

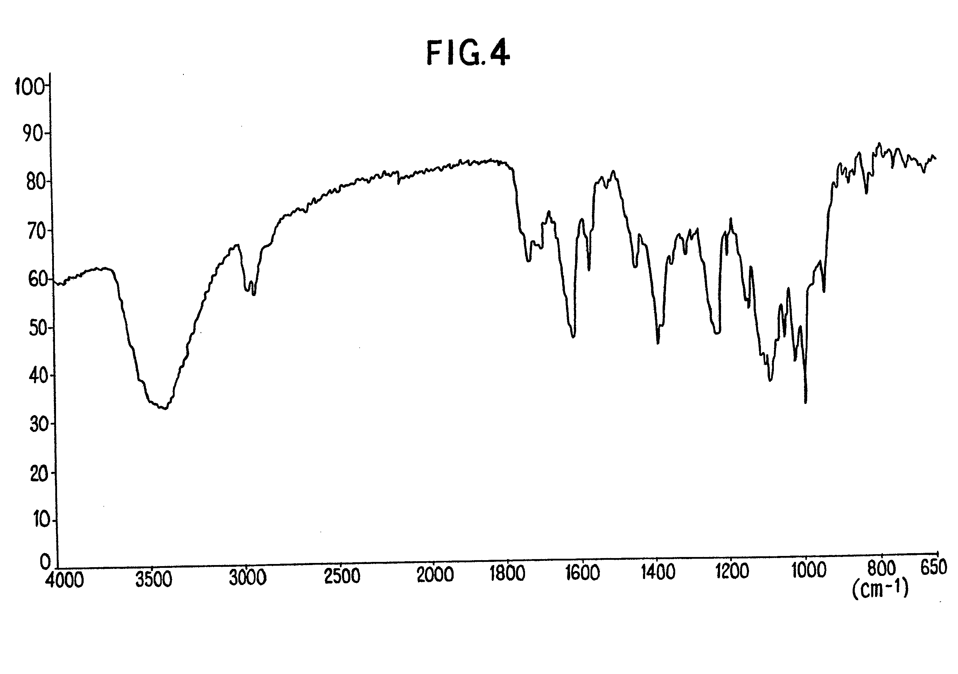

DC—45—$B_1$
(1) Elemental analysis: H:6.15%, C:54.20%
(2) Molecular weight: 895
(3) Melting point:
193—194°C (decomp.)
(4) Ultraviolet absorption spectrum:
As shown in Figure 3 (in 95% ethanol)
(5) Infrared absorption spectrum:
As shown in Figure 4 (KBr tablet method)
(6) Specific rotation:
$[\alpha]_D^{25}=-122.7°$ (c=1.0, $CHCl_3$)
(7) PMR spectrum (in $CDCl_3$; ppm):
1.06 (3H, d, J=6.6); 1.07 (3H, s); 1.25 (3H, d, J=6.6); many peaks are found between 1.40—2.90; 2.14 (3H, s); 2.17 (3H, s); 2.47 (3H, s); 2.60 (3H, broad s); 3.57 (3H, s); 3.65 (3H, s); 3.85 (3H, s) many peaks are found between 3.50—4.20; (4.07 (1H, s); 4.39 (1H, s); many peaks are found between 4.40—5.60; 5.67 (1H, broad s), 6.05 (1H, broad s); 7.53 (1H, s); 14.8 (1H, s)
(8) CMR spectrum (in $CDCl_3$; ppm):
210.9; 203.8; 170.3; 162.1; 152.5; 145.2; 142.3; 135.3; 126.7; 117.0; 114.2; 108.3; 105.3; 99.7; 97.2; 93.7; 85.1; 79.0; 74.6; 71.1; 69.6; 69.3; 68.8; 67.9; 66.3; 64.0; 62.8; 57.3; 55.9; 36.5; 32.2; 28.0; 25.7; 20.9; 20.2; 17.0; 14.7
(9) Solubility:
Soluble in methanol, ethanol, water and chloroform, soluble with difficulty in acetone and ethyl acetate, and insoluble in ether and n-hexane

DC—45—$B_2$
(1) Elemental analysis: H:6.03%, C:54.34%
(2) Molecular weight: 879

2

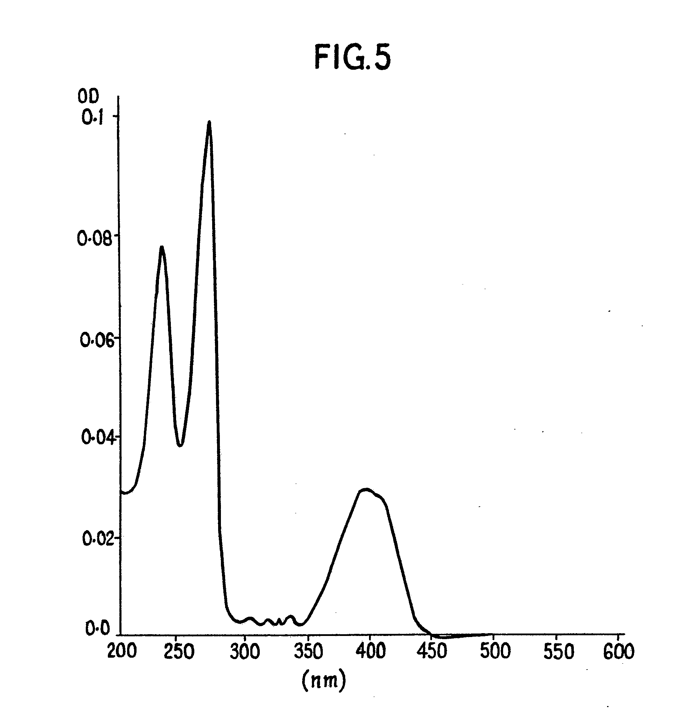

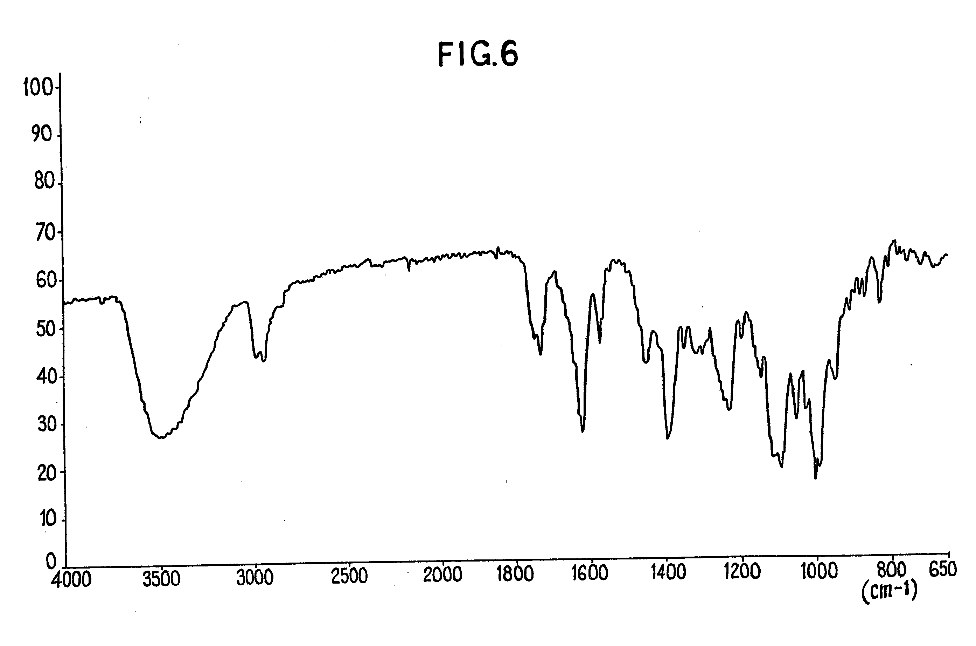

(3) Melting point:
181—182°C (decomp.)
(4) Ultraviolet absorption spectrum:
As shown in Figure 5 (in 95% ethanol)
(5) Infrared absorption spectrum:
As shown in Figure 6 (KBr tablet method)
(6) Specific rotation:
$[\alpha]_D^{25}=-10°$ (c=0.2, ethanol)
(7) PMR spectrum (in $CDCl_3$; ppm):
1.07 (3H, s); many peaks are found between 1.07—1.5; many peaks are found between (1.50—2.80); 2.14 (3H, s); 2.61 (3H, broad s); 286 (1H, d, J=5.7); 2.96 (1H, d, J=5.7); 3.46 (3H, s); 3.63 (3H, s); 3.84 (3H, s); many peaks are found between 3.65—4.20; many peaks are found between 4.40—5.00; many peaks are found between 5.10—5.50; 5.80 (1H, broad s); 7.49 (1H, d, J=1.0); 14.1 (1H, s)
(8) CMR spectrum (in $CDCl_3$; ppm):
202.8; 170.2; 163.1; 151.8; 144.8; 142.9; 135.4; 126.5; 116.8; 114.9; 107.3; 104.6; 101.5; 99.6; 98.0; 94.4; 74.4; 72.5; 71.4; 70.4; 69.1; 68.8; 68.3; 67.9; 67.5; 66.4; 62.9; 62.7; 56.8; 56.5; 48.0; 36.7; 32.3; 25.7; 20.8; 20.3; 18.2; 16.9; 15.5.
(9) Soluble in methanol, ethanol, acetone, ethyl acetate and chloroform, soluble with difficulty in benzene, ether and water, and insoluble in n-hexane; and the salts thereof.

The molecular formula of DC—45—A is $C_{42}H_{52}O_{20}$, the molecular formula of DC—45—$B_1$ is $C_{42}H_{54}O_{21}$ and the molecular formula of DC—45—$B_2$ is $C_{42}H_{54}O_{20}$.
The figures attached are as follows:

| Fig. No. | Ultraviolet absorption spectrum | Infrared absorption spectrum |
|---|---|---|
| 1 | DC—45—A | |
| 2 | | DC—45—A |
| 3 | DC—45—$B_1$ | |
| 4 | | DC—45—$B_1$ |
| 5 | DC—45—$B_2$ | |
| 6 | | DC—45—$B_2$ |

The RF values of the substances DC—45—A, DC—45—$B_1$ and DC—45—$B_2$ as hereinbefore defined are shown in Table 1 and were determined by thin layer chromatography using silica gel (Kieselgel 60 Art. 5721, commercial product of E. Merck, West Germany) and developed for 3 hours at room temperature.

TABLE 1

| No. | Solvent system | Substance | RF |
|---|---|---|---|
| I. | Chloroform/methanol =90:10 (v/v) | DC—45—A | 0.85 |
| | | DC—45—$B_1$ | 0.50 |
| | | DC—45—$B_2$ | 0.45 |
| II. | Ethyl acetate/acetic acid =90:10 (v/v) | DC—45—A | 0.70 |
| | | DC—45—$B_1$ | 0.25 |
| | | DC—45—$B_2$ | 0.35 |
| III. | Upper layer of mixture of 0.1M phosphate buffer (PH:7.0) and ethyl acetate | DC—45—A | 0.70 |
| | | DC—45—$B_1$ | 0.30 |
| | | DC—45—$B_2$ | 0.40 |

According to a still further feature of this invention, there is provided a pharmaceutical composition for human or veterinary use comprising as active ingredient a substance according to the present invention as hereinbefore defined in association with a pharmacologically acceptable carrier or excipient. The composition may be presented in a form suitable for oral, rectal or parenteral administration. Thus, for example, compositions for oral administration may be solid or liquid and may be in the form of granules, tablets, coated tablets, capsules, syrups, emulsions, suspensions or drops, such composition comprising carriers or excipients conventionally used in the pharmaceutical art. Thus, for example, suitable tabletting excipients include lactose, potato and soluble starches and magnesium stearate.

For parenteral administration, the carrier may be a sterile, parenterally acceptable liquid such as sterile water, or a pharmaceutically acceptable oil e.g. arachis oil, contained in ampoules. Composition for rectal administration may take the form of suppositories, the carrier comprising a suppository base.

Advantageously, the composition may be formulated as dosage units, each being adapted to supply a fixed dose of the active ingredient. Tablets, coated tablets, capsules, suppositories and ampoules are examples of suitable dosage unit forms.

The substances of the present invention may if desired be in salt form. For pharmaceutical use the salt will be a physiologically acceptable salt, but other salts may for example be employed in the preparation of the physiologically acceptable salts. Thus, for example, the substances DC—45—A, DC—45—$B_1$ and/or DC—45—$B_2$ may be in the form of salts with an alkali metal, e.g. sodium or potassium; an alkaline earth metal, e.g. calcium or magnesium; or aluminium. The salts may, of course, be prepared by the use of for example the appropriate metal hydroxide or alkoxide in the conventional manner.

The present invention also provides a substance as hereinbefore defined for use as an antibiotic or anti-tumour agent.

The antibiotic activity of the substances DC—45—A, DC—45—$B_1$ and DC—45—$B_2$ are shown in Table 2, the activities being determined by the agar dilution method at pH 7.0.

TABLE 2

Minimum inhibitory concentration ($\mu$g/ml)

| DC—45 | A | B1 | B2 |
|---|---|---|---|
| *Staphylococcus aureus* ATCC 6538P | 0.2 | 50 | 0.4 |
| *Bacillus subtilis* No. 10707 | 0.01 | 12 | 0.01 |
| *Klebsiella pneumoniae* ATCC 10031 | 1.5 | >100 | 1.5 |
| *Salmonella typhosa* ATCC 9992 | 50 | >100 | >100 |
| *Escherichia coli* ATCC 26 | 12 | >100 | 50 |

Acute toxicities of DC—45—A, DC—45—$B_1$ and DC—45—$B_2$:

The $LD_{50}$ values of DC—45—A, DC—45—$B_1$ and DC—45—$B_2$ are respectively 1, 100 and 2 mg/kg (mouse- ip.).

Anti-tumour activities of DC—45—A, DC—45—$B_1$ and DC—45—$B_2$:

(1) Therapeutic effect on Sarcoma 180 ascites tumour:

Mice (male, ddy-strain; weight about 20 g; each group consisting of 6 mice) were used as the test animal for these determinations and Sarcoma 180 ascites tumour ($5 \times 10^6$ cells) was implanted into each mouse under the skin at the armpit. After 24 hours, a phosphate buffered physiological solution of sodium chloride (each dose (0.2 ml) was abdominally administered to each mouse, a given amount of one of substances DC—45—A, DC—45—$B_1$ and DC—45—$B_2$ being contained in each solution so that a different substance is administered to each group of mice. The said phosphate buffer contains NaCl (0.8 g/dl), KCl (0.02 g/dl), $Na_2HPO_4$ (1.15 g/dl) and $KH_2PO_4$ (0.02 g/dl). For comparison purposes, a similar phosphate buffered physiological solution containing mitomycin C (0.2 ml) was abdominally administered to the mice of the control group 24 hours after the implantation of the tumour cells. Table 3 indicates the volume of the tumour (mean value in $mm^3$) and T/C (mean value; the tumour volume of the animal treated with the test compound/the tumour volume of the control animal treated with 0.2 ml of the phosphate buffered solution of sodium chloride), both being determined 7 days after the implantation.

TABLE 3

| Test substance | Dose (mg/kg) | Volume of tumour (mean value) mm$^3$ | T/C |
|---|---|---|---|
| DC—45—A | 0.2 | 775 | 0.55 |
| | 0.1 | 930 | 0.66 |
| | 0.2* | 592 | 0.42 |
| | 0.1* | 846 | 0.60 |
| DC—45—B$_1$ | 75 | 775 | 0.55 |
| | 50 | 846 | 0.60 |
| | 20 | 1057 | 0.75 |
| DC—45—B$_2$ | 1.0 | 493 | 0.35 |
| | 0.5 | 1170 | 0.83 |
| Mitomycin C | 5.6 | 282 | 0.20 |
| Untreated | 0 | 1410 | - - - |

Note:— *Continued for 7 days (once daily)

(2) Therapeutic effect of substances DC—45—A, DC—45—B$_1$ and DC—45—B$_2$ on lymphocytic leukaemia P—338 tumour:

Mice (male; CDF$_1$ strain; weight about 22 g; each group consisting of 5 mice) were used as the test animals.

Lymphocytic leukaemia P—388 tumour (1×10$^6$ cells) was abdominally implanted into each mouse. After 24 hours, a phosphate buffered physiological solution (0.2 ml) was abdominally injected once into each mouse a given amount of one substance being contained in each solution so that a different substance is administered to each group of mice. For comparison purposes, a similar phosphate buffered solution of sodium chloride containing mitomycin C was administered abdominally to each mouse of a further control group. Table 4 indicates the average survival time in days and T/C (mean value of the survival days of the animals treated with the test compound/mean value of the survival days of the control animals treated with 0.2 ml of the phosphate buffered solution of sodium chloride).

TABLE 4

| Substance | Dose (mg/kg) | Survival days | Elongation of life span (T/C) |
|---|---|---|---|
| Untreated | - - - | 9.6±0.9 | - - - |
| DC—45—A | 0.2* | 11.3±0.8 | 1.18 |
| | 0.1* | 13.0±0.9 | 1.35 |
| DC—45—B$_1$ | 50 | 15.5±1.7 | 1.62 |
| | 20 | 14.2±1.5 | 1.48 |
| | 12.5 | 15.0±1.4 | 1.56 |
| DC—45—B$_2$ | 1.0 | 12.0±2.0 | 1.25 |
| | 0.5 | 12.8±2.2 | 1.33 |
| | 0.25 | 12.2±0.8 | 1.27 |
| Mitomycin C | 4.2 | 18.0±1.4 | 1.88 |

Note:— *Continued once daily for 5 days

The substances of the present invention have been prepared by investigating the substances produced by culturing various microorganisms which we have collected from natural sources. Thus the antibiotic substances of the present invention, designated DC—45—A, DC—45—B$_1$ and DC—45—B$_2$,

5

have been isolated from the cultured broth obtained by culturing a microorganism, which we have isolated from the soil in Sapporo City, Japan.

Thus according to a further feature of the present invention there is provided a process for the preparation of the substance DC—45—A, DC—45—B$_1$ and/or DC—45—B$_2$ as hereinbefore defined which comprises culturing the microorganism *Streptomyces bottropensis* (FERM—P No. 5219; NRRL 12051) or a mutant thereof capable of producing the substance DC—45—A, DC—45—B$_1$ and/or DC—45—B$_2$ as hereinbefore defined in a culture medium to accumulate the said substance(s) in the cultured broth and recovering the said substance(s) therefrom.

The characteristics of *Streptomyces bottropensis* (FERM—P No. 5219; NRRL 12051) formerly named *Streptomyces ochraceus* DC—45 are detailed below:—

Taxonomic characteristics:

(a) Morphological characteristics:

Aerial mycelia are well formed and appear to be simply branched and spiral, when the above listed strain is cultured in a conventional medium. Spores are in the form of chains having more than 10 links. The spore surface is smooth. Spores are ellipsoidal (0.4—0.5$\mu \times$0.7—0.8$\mu$). Table 5 indicates the degree of growth, colour on the surface, colour at the back of colonies and colour of soluble pigment, which are observed by culturing the above-titled strain using various media. The colour classification described in Colour Harmony Manual published by Container Corpn. of America is used for colour identification.

## TABLE 5
### Growth on various media

| | Growth | Colour of colonies | | Growth & colour aerial mycelium | Soluble pigment |
|---|---|---|---|---|---|
| | | Surface | Back | | |
| Sucrose–nitrate agar | good, flat | oatmeal (2 ec) | oatmeal (2 ec) | moderate natural string (2 de) | nil |
| Glucose–asparagine agar | good, flat | light rose beige (4 ec) | mustard (2 le) | poor natural (3 dc) | cinnamon (3 lc) |
| Glycerin–asparagine agar | good, raised | shell (3 ca) | shell (3 ca) | nil | nil |
| Starch–inorganic salt agar | moderate, flat | pussy willow gray (5 dc) | sand (3 gc) | moderate white (a) | camel (3 ie) |
| Tyrosin agar | good, flat | yellow tint (1 ba) | ivory tint (2 cb) | nil | nil |
| Nutrient agar | poor, flat | camel (3 ie) | bamboo (2 gc) | nil | camel (3 ie) |
| Yeast–malt extract agar | good, raised | rose beige (4 ge) | mustard (2 le) | poor white (a) | chestnut brown (4 ni) |
| Oatmeal agar | good, raised | oatmeal (2 ec) | oatmeal (2 ec) | good covert gray (2 fe) | nil |
| Peptone–yeast–iron agar | good, flat | oatmeal (2 ec) | oatmeal (2 ec) | nil | clove brown (3 ni) |

0 029 309

(B) Physiological characteristics:

The physiological characteristics of *Streptomyces bottropensis* (FERM—P No. 5219; NRRL 12051) are set out in the following where the results were determined after culturing four 2 weeks at 27°C with the exception that the temperature was determined after 5 days and the actions upon milk and cellulose were determined after one month.

1) Utilization of carbon sources:

| Carbon sources | Assimilability |
|---|---|
| D-arabinose | + |
| D-xylose | ++ |
| D-glucose | ++ |
| D-fructose | ++ |
| Sucrose | ++ |
| Inositol | ++ |
| L-rhamnose | + |
| Raffinose | ++ |
| D-mannit | ++ |

| | | |
|---|---|---|
| 2) | Liquefaction of gelatin: | — |
| 3) | Action upon milk: | |
| | Liquefaction | ± |
| | Coagulation | — |
| | Peptonization | — |
| 4) | Decomposition of cellulose | slightly positive |
| 5) | Hydrolysis of starch | positive |
| 6) | Optimum growth pH | 6.8—7.5 |
| 7) | Optimum growth temperature | 28—382C |
| 8) | Production of tyrosinase | — |
| 9) | Production of melanoid pigment | — |

From these characteristics, the above-mentioned microorganism may be classified into the genus *Streptomyces*. This microorganism closely resembles in morphological and physiological characteristics the species *Streptomyces parvulus* according to the classification by E. Küster [Intern. J. System. Bacteriol., vol. 22, No. 3, page 139 (1972)] because its aerial mycelium is grayish. However, microorganisms of the genus *S. parvulus* are not capable of metabolizing raffinose and thus this microorganism is apparently different from *S. parvulus*. As a result, the present strain was originally designated *S. ochraceus* DC—45 in view of the colour of its vegetative mycelia. As a result of further studies the original designation of the strain as *S. ochraceus* was amended to *S. bottropensis*. The present strain has been deposited with Bikoken (the Fermentation Research Institute), of 1—1—3, Higashi Yatabe-cho, Tsukuba-gun, Ibaraki-ken, Japan as FERM P—5219 and also with the Northern Regional Research Centre, Agricultural Research North Central Region, Peoria, Illinois, U.S.A. as NRRL 12051. The microorganism was filed with the above-mentioned Northern Regional Research Centre on 6 October 1979.

Like other strains of the genus *Streptomyces*, it may be possible to obtain mutant strains by treating the present strain with various mutagens, for example, ultraviolet irradiation, X-ray irradiation, chemicals and the like. Such mutuant strains may also be used for the purpose of this invention when they are capable of producing the substances of the present invention and the use of such microorganisms are regarded as falling within the scope of the present invention.

According to a further feature of the present invention there is provided the microorganism *Streptomyces bottropensis* (FERM—P No. 5219; NRRL 12051). The microorganism is preferably in or on a sterile medium.

Method of fermentation of *Streptomyces bottropensis* (FERM P— No. 5219; NRRL 12051):

Any and all methods for culturing conventional strains of the genus *Streptomyces* may be used for the fermentation of the above-titled strain. Various nutrients as exemplified below may be used for fermentation. For example, as the carbon sources, glucose, starch, dextrin, mannose, fructose, sucrose, lactose, xylose, arabinose, mannitol, molasses and the like may be used solely or in combination, although it is possible to use, depending upon the assimilability of the strain used, various hydrocarbons, alcohols and organic acids.

Inorganic and organic nitrogen containing compounds such as ammonium chloride, ammonium sulfate, ammonium nitrate, sodium nitrate and urea may for example be used as the inorganic and/or organic nitrogen source, although it is possible to use various natural nitrogen sources such as for example peptone, meat extract, yeast extract, dry yeast, corn steep liquor, soybean meal and casamino acid, these substances being used alone or in combination. If desired, various inorganic salts such as for example sodium chloride, potassium chloride, magnesium sulfate, calcium carbonate, potassium monohydrogen phosphate, potassium dihydrogen phosphate, ferrous sulfate, calcium chloride, manganese sulfate, zinc sulfate, copper sulfate and the like may be used. Moreover, for example, vitamin $B_1$, biotin and other trace components may conveniently be used to promote the growth of the microorganism and to enhance the production of the compounds of the present invention.

Liquid culturing procedures, in particular, submerged culturing with stirring, are preferred for the fermentation of the microorganisms here concerned. The fermentation is preferably effected at a temperature of from 25 to 40°C (for example, 28 to 38°C) advantageously as a pH of 4 to 10 (for example 6 to 8) which may for example be adjusted by using aqueous ammonia or ammonium carbonate solution.

In general, the compounds of the present invention may be produced and accumulated in the cultured broth by effecting the liquid culture for one to seven days. The fermentation is, of course, preferably continued until the amount of each or all of the desired substances of the present invention in the cultured broth reach their maximum. The fermentation is then discontinued and the cultured liquor is filtered to separate the microbial cells from the liquor.

The substances of the present invention may be isolated from the filtrate and purified by methods conventionally used for the isolation and purification of metabolic products present in the cultured liquor of microorganisms. For example, a cell-free filtrate having a pH of 6.0 is passed through a column packed with a non-ionic porous resin (e.g. Diaion HP—20, commercial product of Mitsubishi Kasei Kogyo K. K., Tokyo the word 'Diaion being a registered Trade Mark') to absorb the active substance onto the resin, from which the active substance is eluted using, for example, methanol, acetone or ethyl acetate. The eluted fractions are concentrated to dryness. Celite (registered Trade Mark) powders are used to absorb the active substance, and the powders are suspended in a mixture of n-hexane and ethyl acetate (1:1 v/v) on a column packed with silica gel. A mixture of n-hexane and ethyl acetate (1:1 v/v) is passed through the column to remove impurities and the column is then eluted by using a mixture of n-hexane and ethyl acetate (1:5 v/v) to elute the fractions containing the compound designated as DC—45—A. By subsequent elution using ethyl acetate, fractions containing a mixture of the compounds designated DC—45—$B_1$ and DC—45—$B_2$ may be obtained. Fractions containing DC—45—A are concentrated to dryness *in vacuo*. The residue is chromatographed by using a column packed with silica gel which has previously been suspended in chloroform. At first, impurities are removed by passing chloroform through the column and then a mixture of chloroform/methanol (100:1 v/v) is passed through the column so that DC—45—A is eluted. The eluate is concentrated to dryness. The residue is taken up in chloroform and the insoluble substances are removed by centrifugation or filtration. n-Hexane is added to the solution to precipitate DC—45—A. The precipitate is separated and dried to obtain powders of DC—45—A. Other fractions containing DC—45—$B_1$ and DC—45—$B_2$ are concentrated and chromatographed by using a column packed with silica gel previously suspended in chloroform. Chloroform and a mixture of chloroform and methanol (100:1 v/v) are successively passed through the column to remove impurities, and the elution is effected using a mixture of chloroform and methanol (50:1 v/v) to obtain a mixture of DC—45—$B_1$ and DC—45—$B_2$. The eluate is concentrated to dryness and transferred to a column packed with silica gel suspended in a mixed solution of 0.1 M phosphate buffer solution (pH 7.0) and ethyl acetate. The elution is effected by using a similar mixed solvent to that described above so that DC—45—$B_1$ and DC—45—$B_2$ eluted in this order sufficiently separated from each other. Respective fractions are individually collected and concentrated.

The residues are respectively taken up with ethyl acetate and insoluble substances are removed. Acetone is added to the solution of DC—45—$B_1$ and the precipitate is separated and dried to obtain powders of DC—45—$B_2$.

It is also possible to isolate the desired compounds from the microbial cells by extracting the cells with acetone, concentrating the extract to dryness, and treating the dried substances in a similar manner to that described above, namely by silica gel chromatography.

The following non-limiting Examples illustrate the present invention, in which the compounds designated DC—45—A, DC—45—$B_1$ and DC—45—$B_2$ were determined by bioassay using *Bacillus subtilis* No. 10707 or by employing the yellowish colour of the compounds.

Example 1

*Streptomyces bottropensis* (FERM—P No. 5219 NRRL 12051) was used as the seed strain and was inoculated into a seed medium [300 ml; containing KCl (4 g/l), $MgSO_4 7H_2O$ (0.5 g/l), $KH_2PO_4$ (1.5 g/l), ammonium sulfate (5 g/l), sucrose (20 g/l), fructose (10 g/l), glucose (10 g/l), corn steep liquor (5 g/l), $CaCO_3$ (20 g/l); pH 7.0] in a 2 litre Erlenmeyer flask. The fermentation was effected at 30°C for 48 hours with stirring (220 r.p.m.) to obtain a seed culture which was transferred into a 30 litre jar fermenter containing a medium (15 litre; having the composition as hereinafter defined) at a ratio of 5% (v/v). The fermentation was effected at 30°C for 72 hours with shaking and aeration (250 r.p.m.; 15 litre/min) and the pH of the medium was not controlled during the fermentation. Composition of the fermentation medium:

9

Glucose (30 g/l), soluble starch (10 g/l), Farmamedia (cotton seed meal, commercial product of Traders Oil Mill Co., U.S.A.; 10 g/l), $K_2HPO_4$ (1 g/l), $MgSO_4$ $7H_2O$ (1 g/l), NaCl (3 g/l), $CuCO_4$ $5H_2O$ (70 mg/l), $FeSO_4$ $7H_2O$ (10 m/l), $MnCl_2$ $4H_2O$ (8 mg/l), $ZnSO_4$ $7H_2O$ (2 mg/l), $CoCl_2$ $6H_2O$ (0.006 mg/l); pH 7.0 before sterilization, adjusted with NaOH.

After completion of the fermentation, the microbial cells and precipitates were removed from the cultured broth by filtration, resulting in a filtrate (13 l). The filtrate was passed through a column packed with one litre of a non-ionic porous resin (Diaion HP—10, commercial product of Mitsubishi Kasei Kogyo K. K., Tokyo) to adsorb the active substances onto the resin which was then washed with water (about 2 litre), followed by washing with 50% (v/v) methanol (about 2 litre) to remove impurities. After this, elution was effected using methanol. The methanol fraction (about one litre) was concentrated to dryness *in vacuo*, and the dried substance was dissolved in 0.1 M phosphate buffer solution (pH 7.0) and extracted three times with ethyl acetate. The ethyl acetate layer was concentrated and adsorbed onto Cellite to obtain a powder.

Separately, the microbial cells (wet weight: about 200 g) were suspended in acetone (about 5 l) to extract the desired substances. The extract was concentrated to dryness *in vacuo* and the dried material was dissolved in a 0.1M phosphate buffer solution (pH 7.0). The solution was extracted three times with ethyl acetate. The ethyl acetate layer was concentrated and then mixed with Celite to give a powder. The powder was carefully put on a column packed with silica gel (Wako Gel, commercial product of Wako Junyaku K. K., Japan; (500 ml) which had been suspended in n-hexane/ethyl acetate (1:1 v/v) and eluted with a solvent system of n-hexane/ethyl acetate (1:5 v/v) to remove impurities and to obtain fractions containing DC—45—A. By subsequent elution using ethyl acetate, there were obtained fractions containing both DC—45—B$_1$ and DC—45—B$_2$. They were respectively collected, combined and concentrated to dryness. The residue containing DC—45—A was chromatographed by using a column packed with silica gel which had been suspended in chloroform in the following manner:—

Chloroform was passed through the column to remove impurities, and then a mixture of chloroform/methanol (100:1 v/v) was used for elution. DC—45—A was eluted and separated from impurities, the solution obtained then being concentrated. The residue was dissolved in chloroform and the insoluble substances were removed by filtration. n-Hexane was then added to the filtrate. The resultant precipitate was separated off and dried to obtain DC—45—A in the form of a powder (10 mg).

Fractions containing a mixture of DC—45—B$_1$ and DC—45—B$_2$ were chromatographed by passing chloroform through a column, packed with silica gel which had been suspended in chloroform, followed by passing a solvent system of chloroform/methanol (100:1 v/v) through the same so as to remove impurities. After this, a chloroform/methanol mixture (50:1 v/v) was used for elution of a mixture of DC—45—B$_1$ and DC—45—B$_2$. The eluate was concentrated to dryness and the residue was transferred carefully to a column packed with silica gel which had been suspended in the organic layer of a solvent mixture of 0.1M phosphate buffer (pH 7.0) and ethyl acetate. A similar layer was used for elution, and DC—45—B$_2$ and DC—45—B$_1$ were eluted in this order. These fractions were individually collected and concentrated to dryness. The residues were respectively dissolved in ethyl acetate to remove insoluble substances. Acetate was added to the solution containing DC—45—B$_1$, and the precipitate was separated and dried to obtain DC—45—B$_1$ (10 mg) in the form of a powder. n-Hexane was added to the solution containing DC—45—B$_2$ to give a precipitate which was separated and dried to obtain DC—45—B$_2$ in the form of a powder (5 mg).

The physicochemical characteristics of the compounds of the invention thus obtained and their anti-tumour and antibiotic activities are as hereinbefore described.

Example 2

Analogous treatments to those described in Example 1 were carried out except for the use of a fermentation medium having the following composition so that DC—45—A (5 mg), DC—45—B$_1$ (5 mg) and DC—45—B$_2$ (3 mg) were obtained.

Composition of the medium:

Soluble starch (40 g/l), defatted soybean meal (30 g/l), corn steep liquor (5 g/l), $K_2HPO_4$ (0.5 g/l), $MgSO_4$ $7H_2O$ (0.5 g/l), KCl (0.3 g/l), $CaCO_3$ (3 g/l); pH 7.8 before sterilization; adjusted with (NaOH).

**Claims**

1. A substance having antibiotic activity selected from substances designated DC—45—A, DC—45—B$_1$ and DC—45—B$_2$ and respectively possessing the following physioco-chemical characteristics:

DC—45—A:
(1) Elemental analysis:
C:55.11% H:5.74%
(2) Molecular weight: 877

(3) Melting point:
180±3°C (decomp.)
(4) Ultraviolet absorption spectrum:
As shown in Figure 1 (in 50% methanol)
(5) Infrared absorption spectrum:
As shown in Figure 2 (KBr tablet method)
(6) Specific rotation:
$[\alpha]_D^{25} = -15.3°$ (c=1.0, ethanol)
(7) PMR spectrum (in CDCL$_3$; ppm):
1.07 (3H, s); 1.10 (3H, d, J=6.8); 1.24 (3H, d, J=6.5); many peaks are found between 1.40—2.30; 2.14 (3H, s); 2.49 (3H, s); 2.63 (3H, s); many peaks are found between 2.30—2.80; 2.91 (1H, d, J=5.6); 3.00 (1H, d, J=5.6); 3.49 (3H, s); 3.63 (3H, s); 3.85 (3H, s); many peaks are found between 3.60—4.00; 4.18 (1H, s); 4.55 (1H, q, J=6.8); many peaks are found between 4.70—4.90; 5.03 (1H, q, J=6.5); 5.25 (1H, d, J=4.0); 5.39 (1H, d, J=4.0); 5.87 (1H, m); 7.52 (1H, s); 14.1 (1H, s)
(8) CMR spectrum (in CDCl$_3$; ppm):
210.3; 203.0; 170.2; 163.1; 151.7; 144.7; 142.8; 135.4; 126.6; 116.9; 114.8; 107.5; 104.6; 101.5; 99.7; 98.0; 94.8; 79.5; 74.4; 70.2; 69.1; 68.8; 68.3; 67.9; 67.6; 63.8; 62.9; 62.7; 56.7; 56.2; 48.1; 36.7; 31.5; 27.7; 25.7; 20.8; 20.3; 16.8; 14.5.
(9) Solubility:
Soluble in methanol, ethanol, acetone, ethyl acetate and chloroform, soluble with difficulty in benzene, ether and water, and insoluble in n-hexane.

DC—45—B$_1$
(1) Elemental analysis:
C:54.20% H:6.15%
(2) Molecular weight: 895
(3) Melting point:
193—194°C (decomp.)
(4) Ultraviolet absorption spectrum:
As shown in Figure 3 (in 95% ethanol)
(5) Infrared absorption spectrum:
As shown in Figure 4 (KBr tablet method)
(6) Specific rotation:
$[\alpha]_D^{25} = -122.7°$ (c=1.0, CHCl$_3$)
(7) PMR spectrum (in CDCl$_3$; ppm):
1.06 (3H, d, J=6.6); 1.07 (3H, s); 1.25 (3H, d, J=6.6); many peaks are found between 1.40—2.90; 2.14 (3H, s); 2.17 (3H, s); 2.47 (3H, s); 2.60 (3H, broad s); 3.57 (3H, s); 3.65 (3H, s); 3.85 (3H, s); many peaks are found between 3.50—4.20; 4.07 (1H, s); 4.39 (1H, s); many peaks are found between 4.40—5.60; 5.67 (1H, broad s); 6.05 (1H, broad s); 7.53 (1H, s); 14.8 (1H, s)
(8) CMR spectrum (in CDCl$_3$; ppm):
210.9; 203.8; 170.3; 162.1; 152.5; 145.2; 142.3; 135.3; 126.7; 117.0; 114.2; 108.3; 105.3; 99.7; 97.2; 93.7; 85.1. 79.0; 74.6; 71.1; 69.6; 69.3; 68.8; 67.9; 66.3; 64.0; 62.8; 57.3; 55.9; 36.5; 32.2; 28.0; 25.7; 20.9; 20.2; 17.0; 14.7
(9) Solubility:
Soluble in methanol, ethanol, water and chloroform, soluble with difficulty in acetone and ethyl acetate, and insoluble in ether and n-hexane

DC—45—B$_2$
(1) Elemental analysis:
H:6.03% C:54.34%
(2) Molecular weight: 879
(3) Melting point:
181—182°C (decomp.)
(4) Ultraviolet absorption spectrum:
As shown in Figure 5 (in 95% ethanol)
(5) Infrared absorption spectrum:
As shown in Figure 6 (KBr tablet method)
(6) Specific rotation:
$[\alpha]_D^{25} = -10°$ (c=0.2, ethanol)
(7) PMR spectrum (in CDCl$_3$; ppm):
1.07 (3H, s); many peaks are found between 1.07—1.5; many peaks are found between 1.50—2.80); 2.14 (3H, s); 2.61 (3H, broad s); 2.86 (1H, d, J=5.7); 2.96 (1H, d, J=5.7); 3.46 (3H, s); 3.63 (3H, s); 3.84 (3H, s); many peaks are found between 3.65—4.20; many peaks are found between 4.40—5.00; many peaks are found between 5.10—5.50; 5.80 (1H, broad s); 7.49 (1H, d, J=1.0); 14.1 (1H, s)

(8) CMR spectrum (in CDCl$_3$; ppm):

202.8; 170.2; 163.1; 151.8; 144.8; 142.9; 135.4; 126.5; 116.8; 114.9; 107.3; 104.6; 101.5; 99.6; 98.0; 94.4; 74.4; 72.5; 71.4; 70.4; 69.1; 68.8; 68.3; 67.9; 67.5; 66.4; 62.9; 62.7; 56.8; 56.5; 48.0; 36.7; 32.3; 25.7; 20.8; 20.3; 18.2; 16.9; 15.5.

(9) Soluble in methanol, ethanol, acetate, ethyl acetate and chloroform, soluble with difficulty in benzene, ether and water, and insoluble in n-hexane; and the salts thereof.

2. A substance as claimed in claim 1 in substantially pure form.

3. A substance as claimed in claim 1 or claim 2 in the form of a physiologically acceptable salt thereof.

4. A process for the preparation of a substance as defined in claim 1 which comprises culturing the microorganism *Streptomyces bottropensis* (FERM—P No. 5219; NRRL 12051) or a mutant thereof capable of producing a substance as defined in claim 1 in a culture medium to accumulate the said substance in the cultured broth and recovering the said substance therefrom.

5. A pharmaceutical composition for human or veterinary use comprising as active ingredient a substance designated DC—45—A, DC—45—B$_1$ and/or DC—45—B$_2$ as defined in claim 1 or claim 2 or a physiologically acceptable salt thereof in association with a pharmacologically acceptable carrier or excipient.

6. A substance as claimed in any one of claims 1 to 3 for use as an antibiotic or anti-tumour agent.

7. The microorganism *Streptomyces bottropensis* (FERM—P No. 5219; NRRL 12051).

8. A microorganism as claimed in claim 7 in or on a sterile medium.

**Revendications**

1. Une substance ayant une activité antibiotique choisie parmi les substances appelées DC—458—A, DC—45—B$_1$ et DC—45—B$_2$ et possédant respectivement les caractéristiques physico-chimiques suivantes:

DC—45—A:

(1) Analyse élémentaire: C=55,11%, H=5,74%.

(2) Poids moléculaire: 877.

(3) Point de fusion: 180±3°C (décomposition).

(4) Spectre d'absorption ultraviolette:

Comme représenté par la figure 1 (dans du méthanol à 50%).

(5) Spectre d'absorption infrarouge:

Comme représenté par la figure 2 (méthode à la pastille de KBr).

(6) Rotation spécifique:

$[\alpha]_D^{25}=-15,3°$ (c=1,0; éthanol).

(7) Spectre de RMP (dans CDCl$_3$; ppm):

1,07 (3H, s); 1,10 (3H, d, J=6,8); 1,24 (3H, d, J=6,5); on trouve de nombreux pics entre 1,40—2,30; 2,14 (3H, s); 2,49 (3H, s); 2,63 (3H, s); on trouve de nombreux pics entre 2,30—2,80; 2,91 (1H, d, J=5,6); 3,00 (1H, d, J=5,6); 3,49 (3H, s); 3,63 (3H, s); 3,85 (3H, s); on trouve de nombreux pics entre 3,60—4,00; 4,18 (1H, s); 4,55 (1H, q, J=6,8); on trouve de nombreux pics entre 4,70—4,90; 5,03 (1H, q, J=6,5); 5,25 (1H, d, J=4,0); 5,39 (1H, d, J=4,0); 5,87 (1H, m); 7,52 (1H, s); 14,1 (1H, s).

(8) Spectre de RMC (dans CDCl$_3$; ppm):

210,3; 203,0; 170,2; 163,1; 151,7; 144,7; 142,8; 135,4; 126,6; 116,9; 114,8; 107,5; 104,6; 101,5; 99,7; 98,0; 94,8; 79,5; 74,4; 70,2; 69,1; 68,8; 68,3; 67,9; 67,6; 63,8; 62,9; 62,7; 56,7; 56,2; 48,1; 36,7; 31,5; 27,7; 25,7; 20,8; 20,3; 16,8; 14,5.

(9) Solubilité:

Soluble dans le méthanol, l'éthanol, l'acétone, l'acétate d'éthyle et le chloroforme, soluble avec difficulté dans le benzène, l'éther et l'eau et insoluble dans le n-hexane;

DC—45—B$_1$:

(1) Analyse élémentaire: C=54,20%, H=6,15%.

(2) Poids moléculaire: 895.

(3) Point de fusion: 193—194°C (décomposition).

(4) Spectre d'absorption ultraviolette:

Comme représenté par la figure 3 (dans l'éthanol à 95%).

(5) Spectre d'absorption infrarouge:

Comme représenté par la figure 4 (méthode à la pastille de KBr).

(6) Rotation spécifique:
$[\alpha]_D^{25}=-122,7°$ (c=1,0; CHCl$_3$).

(7) Spectre de RMP (dans CDCl$_3$; ppm):
1,06 (3H, d, J=6,6); 1,07 (3H, s); 1,25 (3H, d, J=6,6); on trouve de nombreux pics entre 1,40—2,90; 2,14 (3H, s); 2,17 (3H, s); 2,47 (3H, s); 2,60 (3H, s large); 3,57 (3H, s); 3,65 (3H, s); 3,85 (3H, s); on trouve de nombreux pics entre 3,50—4,20; 4,07 (1H, s); 4,39 (1H, s); on trouve de nombreux pics entre 4,40—5,60; 5,67 (1H, s large); 6,05 (1H, s large); 7,53 (1H, s); 14,8 (1H, s).

(8) Spectre de RMC (dans CDCl$_3$; ppm):
210,9; 203,8; 170,3; 162,1; 152,5; 145,2; 142,3; 135,3; 126,7; 117,0; 114,2; 108,3; 105,3; 99,7; 97,2; 93,7; 85,1; 79,0; 74,6; 71,1; 69,6; 69,3; 68,8; 67,9; 66,3; 64,0; 62,8; 57,3; 55,9; 36,5; 32,2; 28,0; 25,7; 20,9; 20,2; 17,0; 14,7.

(9) Solubilité:
Soluble dans le méthanol, l'éthanol, l'eau et le chloroforme, soluble avec difficulté dans l'acétone et l'acétate d'éthyle et insoluble dans l'éther et le n-hexane;

DC—45—B$_2$:
(1) Analyse élémentaire: C=54,34%, H=6,03%.
(2) Poids moléculaire: 879.
(3) Point de fusion: 181—182°C (décomposition).
(4) Spectre d'absorption ultraviolette:
Comme représenté par la figure 5 (dans l'éthanol à 95%).
(5) Spectre d'absorption infrarouge:
Comme représenté par la figure 6 (méthode à la pastille de KBr).
(6) Rotation spécifique:
$[\alpha]_D^{25}=-10°$ (c=0,2 éthanol).
(7) Spectre de RMP (dans CDCl$_3$; ppm):
1,07 (3H, s); on trouve de nombreux pics entre 1,07—1,5; on trouve de nombreux pics entre 1,50—2,80; 2,14 (3H, s); 2,61 (3H, s large); 2,86 (1H, d, J=5,7); 2,96 (1H, d, J=5,7); 3,46 (3H, s); 3,63 (3H, s); 3,84 (3H, s); on trouve de nombreux pics entre 3,65—4,20; on trouve de nombreux pics entre 4,40—5,00; on trouve de nombreux pics entre 5,10—5,50; 5,80 (1H, s large); 7,49 (1H, d, J=1,0); 14,1 (1H, s).
(8) Spectre de RMC (dans CDCl$_3$; ppm):
202,8; 170,2; 163,1; 151,8; 144,8; 142,9; 135,4; 126,5; 116,8; 114,9; 107,3; 104,6; 101,5; 99,6; 98,0; 94,4; 74,4; 72,5; 71,4; 70,4; 69,1; 68,8; 68,3; 67,9; 67,5; 66,4; 62,9; 62,7; 56,8; 56,6; 48,0; 36,7; 32,3; 25,7; 20,8; 20,3; 18,2; 16,9; 15,5.
(9) Soluble dans le méthanol, l'éthanol, l'acétone, l'acétate d'éthyle et le chloroforme, soluble avec difficulté dans le benzène, l'éther et l'eau et insoluble dans le n-hexane; et leurs sels.

2. Une substance telle que revendiquée dans la revendication 1, sous une forme pratiquement pure.

3. Une substance telle que revendiquée dans la revendication 1 ou la revendication 2 sous forme d'une de ses sels physiologiquement acceptables.

4. Un procédé de préparation d'une substance telle que définie dans la revendication 1, qui comprend la culture du micro-organisme *Streptomyces bottropensis* (FERM—P n° 5219; NRRL 12051) ou d'un de ses mutants capables de produire une substance telle que définie dans la revendication 1 dans un milieu de culture pour accumuler ladite substance dans le bouillon cultivé et la récupération de ladite substance de ce bouillon.

5. Une composition pharmaceutique pour l'utilisation humaine ou vétérinaire, comprenant comme ingrédient actif une substance appelée DC—45—A, DC—45—B$_1$, et/ou DC—45—B$_2$ telle que définie dans la revendication 1 ou la revendication 2 ou un de leurs sels physiologiquement acceptables en association avec un véhicule ou excipient pharmacologiquement acceptables.

6. Une substance telle que revendiquée dans l'une quelconque des revendications 1 à 3 pour l'utilisation comme agent antibiotique ou antitumoral.

7. Micro-organisme *Streptomyces bottropensis* (FERM—P n° 5219; NRRL 12051).

8. Un micro-organisme tel que revendiqué dans la revendication 7 dans ou sur un milieu stérile.

**Patentansprüche**

1. Eine Substanz mit antibiotischer Wirksamkeit, ausgewählt aus den als DC—45—A, DC—45—B$_1$ und DC—45—B$_2$ bezeichneten Substanzen bzw. mit folgenden physiko-chemischen Eigenschaften:

DC—45—A:
(1) Elementaranalyse:
C:55,11% H:5,74%

(2) Molekulargewicht: 877

(3) Schmelzpunkt:
$180\pm3°C$ (Zers.)

(4) Ultraviolett-Absorptionsspektrum:
wie in Fig. 1 gezeigt (in 50% Methanol)

(5) Infrarot-Absorptionsspektrum:
wie in Fig. 2 gezeigt (KBr-Tablettenmethode)

(6) Spezifische Drehung:
$[\alpha]_D^{25}=-15,3°$ (c=1,0, Äthanol)

(7) PMR-Spektrum (in $CDCl_3$; ppm):
1,07 (3H, s); 1,10 (3H, d, J=6,8); 1,24 (3H, d, J=6,5); viele Spitzen werden gefunden zwischen 1,40—2,30; 2,14 (3H, s); 2,49 (3H, s); 2,63 (3H, s); viele Spitzen werden gefunden zwischen 2,30—2,80; 2,91 (1H, d, J=5,6); 3,00 (1H, d, J=5,6); 3,49 (3H, s); 3,63 (3H, s); 3,85 (3H, s); viele Spitzen werden gefunden zwischen 3,60—4,00; 4,18 (1H, s); 4,55 (1H, q, J=6,8); viele Spitzen werden gefunden zwischen 4,70—4,90; 5,03 (1H, q, J=6,5); 5,25 (1H, d, J=4,0); 5,39 (1H, d, J=4,0); 4,87 (1H, m); 7,52 (1H, s); 14,1 (1H, s)

(8) CMR-Spektrum (in $CDCl_3$; ppm):
210,3; 203,0; 170,2; 163,1; 151,7; 144,7; 142,8; 135,4; 126,6; 116,9; 114,8; 107,5; 104,6; 101,5; 99,7; 98,0; 94,8; 79,5; 74,4; 70,2; 69,1; 68,8; 68,3; 67,9; 67,6; 63,8; 62,9; 62,7; 56,7; 56,2; 48,1; 36,7; 31,5; 27,7; 25,7; 20,8; 20,3; 16,8; 14,5.

(9) Löslichkeit:
Löslich in Methanol, Äthanol, Aceton, Äthylacetat und Chloroform, mit Schwierigkeiten löslich in Benzol, Äther und Wasser und unlöslich in n-Hexan.

DC—45—B$_1$

(1) Elementaranalyse:
C:54,20% H:6,15%

(2) Molekulargewicht: 895

(3) Schmelzpunkt:
193—194°C (Zers.)

(4) Ultraviolett-Absorptionsspektrum:
wie in Fig. 3 gezeigt (in 95% Äthanol)

(5) Infrarot-Absorptionsspektrum:
wie in Fig. 4 gezeigt (KBr-Tablettenmethode)

(6) Spezifische Drehung:
$[\alpha]_D^{25}=-122,7°$ (c=1,0, $CHCl_3$)

(7) PMR-Spektrum (in $CDCl_3$; ppm):
1,06 (3H, d, J=6,6); 1,07 (3H, s); 1,25 (3H, d, J=6,6); viele Spitzen werden gefunden zwischen 1,40—2,90; 2,14 (3H, s); 2,17 (3H, s); 2,47 (3H, s); 2,60 (3H, breites s); 3,57 (3H, s); 3,65 (3H, s); 3,85 (3H, s); viele Spitzen werden gefunden zwischen 3,50—4,20; 4,07 (1H, s); 4,39 (1H, s); viele Spitzen werden gefunden zwischen 4,40—5,60; 5,67 (1H, breites s); 6,05 (1H, breites s); 7,53 (1H, s); 14,8 (1H, s)

(8) CMR-Spektrum (in $CDCl_3$; ppm):
210,9; 203,8; 170,3; 162,1; 152,5; 145,2; 142,3; 135,3; 126,7; 117,0; 114,2; 108,3; 105,3; 99,7; 97,2; 93,7; 85,1; 79,0; 74,6; 71,1; 69,6; 69,3; 68,8; 67,9; 66,3; 64,0; 62,8; 57,3; 55,9; 36,5; 32,2; 28,0; 25,7; 20,9; 20,2; 17,0; 14,7

(9) Löslichkeit:
Löslich in Methanol, Äthanol, Wasser und Chloroform, mit Schwierigkeiten löslich in Aceton und Äthylacetat und unlöslich in Äther und n-Hexan

DC—45—B$_2$

(1) Elementaranalyse:
H:6,03% C:54,34%

(2) Molekulargewicht: 879

(3) Schmelzpunkt:
181—182°C (Zers.)

(4) Ultraviolett-Absorptionsspektrum:
wie in Fig. 5 gezeigt (in 95% Äthanol)

(5) Infrarot-Absorptionsspektrum:
wie in Fig. 6 gezeigt (KBr-Tablettenmethode)

(6) Spezifische Drehung:
$[\alpha]_D^{25}=-10°$ (c=0,2, Äthanol)

(7) PMR-Spektrum (in $CDCl_3$; ppm):
1,07 (3H, s) viele Spitzen werden gefunden zwischen 1,07—1,5; viele Spitzen werden gefunden zwischen 1,50—2,80; 2,14 (3H, s); 2,61 (3H, breites s); 2,86 (1H, d, J=5,7); 2,96 (1H, d, J=5,7); 3,46 (3H, s); 3,63 (3H, s); 3,84 (3H, s); viele Spitzen werden gefunden zwischen 3,65—4,20; viele Spitzen werden gefunden zwischen 4,40—5,00; viele Spitzen werden gefunden zwischen 5,10—5,50; 5,80 (1H, breites s); 7,49 (1H, d, J=1,0); 14,1 (1H, s)

(8) CMR-Spektrum (in CDCl$_3$; ppm):
202,8; 170,2; 163,1; 151,8; 144,8; 142,9; 135,4; 126,5; 116,8; 114,9; 107,3; 104,6; 101,5; 99,6; 98,0; 94,4; 74,4; 72,5; 71,4; 70,4; 69,1; 68,8; 68,3; 67,9; 67,5; 66,4; 62,9; 62,7; 56,8; 56,5; 48,0; 36,7; 32,3; 25,7; 20,8; 20,3; 18,2; 16,9; 15,5.

(9) Löslich in Methanol, Äthanol, Aceton, Äthylacetat und Chloroform, mit Schwierigkeiten löslich in Benzol, Äther und Wasser und unlöslich in n-Hexan, und deren Salze.

2. Eine Substanz nach Anspruch 1 in im wesentlichen reiner Form.

3. Eine Substanz nach Anspruch 1 oder 2 in der Form eines physiologisch annehmbaren Salzes.

4. Ein Verfahren zum Gewinnen einer Substanz nach Anspruch 1, dadurch gekennzeichnet, daß man den Mikroorganismus *Streptomyces bottropensis* (FERM—P No. 5219; NRRL 12051) oder eine zur Herstellung einer Substanz nach Anspruch 1 befähigte Mutante desselben in einem Kulturmedium züchtet, um besagte Substanz in der Kulturbrühe anzusammeln, und die besagte Substanz aus letzterer isoliert.

5. Eine pharmazeutische Zusammensetzung für human- oder veterinärmedizinischen Gebrauch, dadurch gekennzeichnet, daß sie als aktiven Bestandteil eine als DC—45—A, DC—45—B$_1$ und/oder DC—45—B$_2$ bezeichnete Substanz nach Anspruch 1 oder 2 oder ein physiologisch annehmbares Salz derselben in Verbindung mit einem pharmakologisch annehmbaren Träger oder Excipienten enthält.

6. Eine Substanz nach einem der Ansprüche 1 bis 3 zur Verwendung als antibiotisches Mittel oder Antitumormittel.

7. Der Mikroorganismus *Streptomyces bottropensis* (FERM—P No. 5219; NRRL 12051.

8. Ein Mikroorganismus nach Anspruch 7 in oder auf einem sterilen Medium.

# FIG.1

FIG.2

# FIG.3

# FIG.4

# FIG.5

FIG.6